(19) Europäisches Patentamt
European Patent Office
Office européen des brevets

(11) EP 2 840 135 B1

(12) EUROPEAN PATENT SPECIFICATION

(45) Date of publication and mention of the grant of the patent:
**01.05.2019 Bulletin 2019/18**

(51) Int Cl.:
*C12N 9/14* (2006.01) *C12P 7/46* (2006.01)

(21) Application number: **14158884.8**

(22) Date of filing: **11.03.2014**

(54) **Cis-epoxysuccinate hydrolase-encoding gene, polypeptide encoded by the gene, and related application thereof**

Cis-Epoxysuccinat-Hydrolase-kodierendes Gen, von dem Gen kodiertes Polypeptid und entsprechende Anwendung dafür

Gène d'encodage d'hydrolase de cis-époxysuccinate, polypeptide codée par ce gène et son application

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.08.2013 CN 201310369774**

(43) Date of publication of application:
**25.02.2015 Bulletin 2015/09**

(73) Proprietor: **Hangzhou Bioking Biochemical Engineering Co., Ltd.**
**Hangzhou 311106 (CN)**

(72) Inventors:
- **Zhang, Jianguo**
**311106 Hangzhou (CN)**
- **Xie, Zhipeng**
**311106 Hangzhou (CN)**
- **Cheng, Yongqing**
**311106 Hangzhou (CN)**
- **Pan, Haifeng**
**311106 Hangzhou (CN)**
- **Bao, Wenna**
**311106 Hangzhou (CN)**

(74) Representative: **Jacob, Reuben Ellis et al**
**Maucher Jenkins**
**26 Caxton Street**
**London SW1H 0RJ (GB)**

(56) References cited:

- **DATABASE GenBank [Online] NCBI; 8 March 2014 (2014-03-08), Zhang,J., Xie,Z., Cheng,Y. and Pan,H.: "A novel cis-epoxysuccinate hydrolase from Klebsiella sp. BK-58: its isolation, purification, cloning and reaction mechanism analysis", XP002733853, Database accession no. KF977193**
- **ZHIQIANG LIU ET AL: "Cloning, sequencing, and expression of a novel epoxide hydrolase gene from Rhodococcus opacus in Escherichia coli and characterization of enzyme", APPLIED MICROBIOLOGY AND BIOTECHNOLOGY, SPRINGER, BERLIN, DE, vol. 74, no. 1, 17 October 2006 (2006-10-17), pages 99-106, XP019472547, ISSN: 1432-0614**
- **ZIQIANG WANG ET AL: "Expression and production of recombinant cis-epoxysuccinate hydrolase in Escherichia coli under the control of temperature-dependent promoter", JOURNAL OF BIOTECHNOLOGY, vol. 162, no. 2-3, 1 December 2012 (2012-12-01), pages 232-236, XP055157588, ISSN: 0168-1656, DOI: 10.1016/j.jbiotec.2012.09.011**
- **ZIQIANG WANG ET AL: "Purification and characterization of a cis-epoxysuccinic acid hydrolase from Nocardia tartaricans CAS-52, and expression in Escherichia coli", APPLIED MICROBIOLOGY AND BIOTECHNOLOGY, vol. 97, no. 6, 1 March 2013 (2013-03-01), pages 2433-2441, XP055157591, ISSN: 0175-7598, DOI: 10.1007/s00253-012-4102-4**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 840 135 B1

Remarks:

The file contains technical information submitted after the application was filed and not included in this specification

## Description

## FIELD OF THE INVENTION

The present invention relates to microbiological genetic engineering and provides a novel *cis*-epoxysuccinate hydrolase-encoding gene and a polypeptide encoded by the gene. The invention further provides polynucleotides for encoding the polypeptide, a recombinant expression vector and a recombinant microbial strain for expressing the polypeptide, and a method for producing L(+)-tartaric acid or its salts.

## BACKGROUND OF THE INVENTION

L(+)-Tartaric acid [(2R,3R)-2,3-dihydroxy-1,4-butanedioic acid] is a natural organic acid abundant in some fruits, such as grapes and tamarind. It is widely used in various industries, such as food, medicine, construction, chemical, textile, electroplating industry and the like. Demand for L(+)-tartaric acid has been increasing progressively year by year. In the past, L(+)-tartaric acid was mainly extracted from tartar which was by-product of grape wine. At present, L(+)-tartaric acid or its salts are mainly produced by catalysis of *cis*-epoxysuccinate hydrolase expressed by microorganisms which typically includes the following steps: first, adding water to a raw material *cis*-butenedioic anhydride to obtain *cis*-butenedioic acid; second, triggering a reaction between *cis*-butenedioic acid and hydrogen peroxide by using tungstic acid or its salts as catalyst to obtain *cis*-epoxysuccinic acid or its salts; and then, hydrolyzing *cis*-epoxysuccinic acid or its salts using *cis*-epoxysuccinate hydrolase or microorganisms having *cis*-epoxysuccinate hydrolase to obtain L(+)-tartaric acid or its salts.

In 1974, Eiji Sato *et al.* synthesized L(+)-tartaric acid using an enzymatic method, which involves using a chemosynthetic *cis*-epoxysuccinic acid as a substrate, and hydrolyzing the substrate to produce L(+)-tartaric with a microorganism having *cis*-epoxysuccinate hydrolase. This method hardly produces any by-products.

In 1976, Kamatani *et al.* described a method for producing L(+)-tartaric acid through microbial transformation of *cis*-epoxysuccinic acid in U.S. Patent No. 4028185A, U.S. Patent No. 4013509A and U.S. Patent No. 4011135A. This method made mass production of L(+)-tartaric acid practicable.

In 2006, Liu *et al.* described cloning, sequencing, and expression of an epoxide hydrolase gene from *Rhodococcus opacus* in *Escherichia coli* and characterization of the enzyme.

In 2012, Wang *et al.* described expression and production of recombinant *cis*-epoxysuccinate hydrolase in *Escherichia coli* under the control of a temperature-dependent promoter.

Current known microorganisms for producing L(+)-tartaric acid or its salts mainly include: *Achromobacter, Acetobacter, Agrobacterium, Alcaligenes, Acinetobacter, Nocardia, Rhizobium, Rhodococcus, Pseudomonas* and *Corynebacterium.*

It is generally recognized that, microbial transformation for producing L(+)-tartaric acid have advantages of good stereospecificity, quick enzymatic reaction, high optical purity and high yield of products, and simple separation and purification of reaction products. However, expression level of *cis*-epoxysuccinate hydrolase is low because of extremely complex intrinsic enzyme systems in wild-type microorganisms, and the activity of *cis*-epoxysuccinate hydrolase is restricted by various intracellular and extracellular factors, which leads to a low production efficiency of L(+)-tartaric acid. By constructing genetically engineered bacteria having *cis*-epoxysuccinate hydrolase-gene using genetic engineering technology, a high expression level of *cis*-epoxysuccinate hydrolase can be achieved, which also allows for industrial production of L(+)-tartaric acid.

## SUMMARY OF THE INVENTION

A main object of the present invention is to overcome a defect of low production efficiency of L(+)-tartaric acid caused by low expression level of *cis*-epoxysuccinate hydrolase, and to provide a novel *cis*-epoxysuccinate hydrolase-encoding gene. A technical problem to be solved is how to achieve a high expression level of *cis*-epoxysuccinate hydrolase encoded by the gene, so as to improve production efficiency of L(+)-tartaric acid.

According to a first aspect of the invention, there is provided a *cis*-epoxysuccinate hydrolase-encoding gene, characterized in that: the *cis*-epoxysuccinate hydrolase-encoding gene includes a segment having a nucleotide sequence as shown in SEQ ID NO. 1.

According to a second aspect of the invention, there is provided a polypeptide, characterized in that the polypeptide has an amino acid sequence as shown in SEQ ID NO. 2.

According to a third aspect of the invention, there is provided a polypeptide, characterized in that: the polypeptide has an amino acid sequence that is derived based on the amino acid sequence as shown in SEQ ID NO. 2 through substitution, deletion or addition of amino acids, the polypeptide is more than 50% homologous to that shown in SEQ ID NO. 2, wherein the polypeptide exhibits *cis*-epoxysuccinate hydrolase activity, and the *cis*-epoxysuccinate hydrolase activity level is at least 95% of that of the complete amino acid sequence SEQ ID NO. 2.

According to a fourth aspect of the invention, there is provided a polynucleotide, characterized in that it has a nucleotide sequence for encoding the polypeptide of the third aspect of the invention.

According to a fifth aspect of the invention, there is provided recombinant expression vector or a recombinant microbial strain that is usable for expressing the polypeptide of any of the second or third aspects of the invention.

According to a sixth aspect of the invention, there is provided a method for preparing L(+)-tartaric acid or its salts using the polypeptide of the second or third aspects of the invention, characterized in that the L(+)-tartaric acid or its salts are produced by hydrolysis of *cis*-epoxysuccinic acid or salts thereof under catalysis of the said polypeptide.

The nucleotide sequence of the *cis*-epoxysuccinate hydrolase-encoding gene may be derived from a separated and purified strain of *Klebsiella* sp., preferably a strain of *Klebsiella* sp. BK-58 deposited at China General Microbiological Culture Collection Center (CGMCC, located at No. 3, No. 1 courtyard, Beichen west road, Chaoyang district, Beijing, China) on 12 June 2011, having a deposit Number of CGMCC No. 4949.

The above-mentioned nucleotide sequence (genome DNA, cDNA or RNA) of *cis*-epoxysuccinate hydrolase-encoding gene is more than 70% homologous to the nucleotide sequence as shown in SEQ ID NO. 1, preferably 80% homologous, and more preferably 90% homologous. Specifically, the nucleotide sequence of the *cis*-epoxysuccinate hydrolase-encoding gene provided by the present invention can be an allele mutant of the nucleotide sequence as shown in SEQ ID NO.1, a nucleotide sequence separated and obtained from another species, or obtained by truncating, deleting, substituting or adding one or several nucleotides from or to the nucleotide sequence as shown in SEQ ID NO. 1. Homology between the sequences can be determined by standard alignment techniques, such as by using Clustal X.

The nucleotide sequence of the *cis*-epoxysuccinate hydrolase-encoding gene according to the present invention is a nucleotide sequence containing all or a part of the sequence as shown in SEQ ID NO. 1.

The above-mentioned "nucleotide sequence containing a part of the sequence as shown in SEQ ID NO. 1" means that the nucleotide sequence contains more than 100 consecutive nucleotides identical to those as shown in SEQ ID NO. 1. This may also means that an amino-polypeptide encoded thereby has a similar *cis*-epoxysuccinate hydrolase activity as an amino-polypeptide (a complete amino acid sequence as shown in SEQ ID NO. 2) encoded by the complete sequence as shown in SEQ ID NO. 1.

Preferably, the amino-polypeptide encoded by the "nucleotide sequence containing a part of the sequence as shown in SEQ ID NO. 1" has a *cis*-epoxysuccinate hydrolase activity level no less than 80% of that of the amino-polypeptide (SEQ ID NO. 2) encoded by the complete sequence as shown in SEQ ID NO. 1, and more preferably has 100% of the *cis*-epoxysuccinate hydrolase activity level of SEQ ID NO. 2.

The nucleotide sequence may be a recombinant nucleotide sequence, which contains the above-mentioned nucleotide sequence of the *cis*-epoxysuccinate hydrolase-encoding gene adjoining one or more regulatory sequences from homologous or heterologous microorganisms.

The regulatory sequences contain one or more sequences selected from promoter sequence, secretion signal sequence, and termination signal sequence.

The present invention further provides a vector containing the nucleotide sequence of the *cis*-epoxysuccinate hydrolase-encoding gene and may optionally further containing one or more regulatory sequences obtained from homologous or heterologous microorganisms.

The "vector" may be any biochemical frameworks which can be used to transduce or transfect nucleotide sequences into host cells. The vector may be one or more selected from plasmid, virus, phage, liposome, and cationic vesicle. The vector can contain one or more of the regulatory sequences. Preferably, the vector is plasmid.

The host cell used in the present invention is preferably a recombinant host cell which is transformed by the nucleotide sequence or vector.

The "recombinant host cell which is transformed by the nucleotide sequence or vector" refers to a host cell which does not itself have the above-mentioned nucleotide sequence of the *cis*-epoxysuccinate hydrolase-encoding gene or vector, but can carry the nucleotide sequence or the vector after transformation.

The above-mentioned host cell can express the *cis*-epoxysuccinate hydrolase-encoding gene or vector carrying the same, and produce the amino acid sequence encoded by the nucleotide sequence or vector. The nucleotide sequence in the present invention can be integrated to the genome of the selected host cell or exist as an episomal vector in the host cell.

For convenience, the recombinant host cell used in the present invention includes microorganisms, preferably bacteria or fungi, including yeast. After reconstruction and optimization, the recombinant host cell can express *cis*-epoxysuccinate hydrolase at a high level.

The above-mentioned "express at a high level" means to achieve overexpression by controlling the nucleotide sequence via neighboring regulatory sequences.

The present invention further provides a separated and purified amino acid sequence of *cis*-epoxysuccinate hydrolase, which is encoded by the aforementioned nucleotide sequence, and/or expressed by the aforementioned recombinant host cell.

The *cis*-epoxysuccinate hydrolase according to the present invention may have a molecular mass of 25-35 kDa, and preferably of about 30 kDa (a theoretical value is 30.124 kDa).

The amino acid sequence or polypeptide of the *cis*-epoxysuccinate hydrolase may be expressed by the recombinant host cell through extracellular or intracellular expression and/or secretory expression.

In one example, the amino acid sequence of the *cis*-epoxysuccinate hydrolase according to the present invention is more than 50% homologous to that shown in SEQ ID NO. 2, and preferably more than 70% homologous, and more preferably more than 90% homologous.

The amino acid sequence of the *cis*-epoxysuccinate hydrolase according to the present invention contains all or a part (more than 50 amino acids, preferably more than 100) of the amino acid sequence as shown in SEQ ID NO. 2. Its *cis*-epoxysuccinate hydrolase activity level is at least 95%. That is, the amino acid sequence of the *cis*-epoxysuccinate hydrolase according to the present invention can maintain its activity level even after substitution, deletion or addition by one, several (such as two, three, four, five, six, seven, eight, nine) or multiple (such as ten or more than ten) amino acids. The molecular mass of the *cis*-epoxysuccinate hydrolase or its partial sequence may be about 30 kDa or lower or higher.

The *cis*-epoxysuccinate hydrolase of the present invention can be authenticated using well-known enzyme experiments, such as by hydrolyzing the *cis*-epoxysuccinate or its salts into L(+)-tartaric acid or its salts. Microorganisms are cultured in appropriate culture medium to produce enzymes. Cultures or microbial cells separated therefrom can be used for testing enzymatic activity thereof.

In an appropriate culture medium, wild microorganisms or recombinant cells are induced to produce *cis*-epoxysuccinate hydrolase. The target *cis*-epoxysuccinate hydrolase can then be separated using conventional techniques such as column chromatography. "Active sites" on the amino acid sequence can be detected, and the DNA sequence of the *cis*-epoxysuccinate hydrolase-encoding gene can be obtained from the above-mentioned wild microorganisms or recombinant cells.

The DNA sequence of the *cis*-epoxysuccinate hydrolase-encoding gene is obtained based on the N-terminal and C-terminal sequences of the *cis*-epoxysuccinate hydrolase purified from the microorganisms.

Degenerate primers may be designed according to the N-terminal and C-terminal sequences of the purified *cis*-epoxysuccinate hydrolase, and the PCR (polymerase chain reaction) product of about 0.8 kb, containing *cis*-epoxysuccinate hydrolase-encoding sequence, is inserted into vector pUCm-T through PCR technology. A DNA sequence of the whole inserted fragment can be detected using common sequencing method known in the art.

A PCR product containing the *cis*-epoxysuccinate hydrolase-encoding sequence is obtained by using primers containing Nde I and BamH I restriction enzyme recognition sites through PCR technology and inserted into vector pUCm-T. Nde I and BamH I restriction enzymes are used to digest the vector pUCm-T containing the *cis*-epoxysuccinate hydrolase-encoding sequence and the cleaved fragment is inserted into the corresponding restriction enzyme sites of the vector pET-22b(+). A DNA sequence of the whole inserted fragment is detected through common sequencing technology known in the art.

The constructed expression vector pET-22b(+) inserted with *cis*-epoxysuccinate hydrolase-encoding sequences as described above are expressed in host cells *Escherichia coli.* The host cells *E. coli* containing vector pET-22b(+) inserted with *cis*-epoxysuccinate hydrolase-encoding sequence are cultured in an appropriate medium containing corresponding antibiotic, such as LB medium, to keep the plasmid pET-22b(+) inserted with *cis*-epoxysuccinate hydrolase-encoding sequence continuously present in the host cells which are then collected to detect *cis*-epoxysuccinate hydrolase activity.

Expression of the *cis*-epoxysuccinate hydrolase gene can be by media other than *E. coli.* For example, DNA fragments of the *cis*-epoxysuccinate hydrolase-encoding gene can be expressed conveniently in bacteria or fungi, including yeast. In order to allow the above-mentioned gene to express in bacteria, fungi and yeast, the *cis*-epoxysuccinate hydrolase-encoding gene can be (over) expressed in selected host cells by controlling neighboring DNA sequences, such as promoter, terminator, and upstream activating sequence.

The DNA sequence of the encoding gene of *cis*-epoxysuccinate hydrolase can be appropriately modified, thus allowing *cis*-epoxysuccinate hydrolase to secrete (through extracellular expression) in the culture medium of the host cells. Usually, such modification can be achieved by inserting a DNA sequence for coding a leader sequence. The leader sequence allows for secretory expression of *cis*-epoxysuccinate hydrolase in selected host cells through a secretion mechanism and allows for re-naturation of the *cis*-epoxysuccinate hydrolase in the culture medium of the host cells.

The following examples provide culture conditions (such as medium and temperature) of host cells for expressing *cis*-epoxysuccinate hydrolase.

In one example, there is a method for hydrolyzing *cis*-epoxysuccinic acid or its salts using the recombinant host cell or the amino acid sequence of *cis*-epoxysuccinate hydrolase. The salts of the *cis*-epoxysuccinic acid are formed by *cis*-epoxysuccinic acid and various cations, including but not limited to ammonium ion, potassium ion, magnesium ion, and calcium ion.

The *cis*-epoxysuccinate hydrolase can be used in the forms described as follows: the cells after the above-mention culturing, with or without dialysis treatment; the purified enzyme; the cell extracts (part of substances of the host cells obtained after one or more rounds of centrifugation and extraction). The enzyme can also be used in combination with other enzymes in any form as mentioned above. *Cis*-epoxysuccinate hydrolase is secreted extracellularly and renatured in the culture medium. That is, the enzyme can be used in combination with or independent of one or more enzymes in the form of a crude enzyme or a partial (or total) fine enzyme.

The present invention also provides a method for producing L(+)-tartaric acid or its salts using the genetically engineered bacteria according to the present invention. L(+)-tartaric acid or its salts can be obtained by adding genetically engineered bacteria according to the present invention into *cis*-epoxysuccinic acid or a solution of its salt and then performing enzyme-catalyzed reaction at an appropriate temperature. In one example, the concentration of *cis*-epoxysuccinic acid or its salts in the solution is 0.1 - 2 mol/L, or preferably 0.5 - 1.5 mol/L, or more preferably 0.8 - 1.2 mol/L; the pH thereof is 4.5 - 11.0, or preferably 6.0 - 9.5, or more preferably 8.0 - 9.0; and reaction temperature is 10 - 55°C, or preferably 20 - 45°C, or more preferably 32 - 40°C.

The *cis*-epoxysuccinate hydrolase-encoding gene may be cloned from a strain of *Klebsiella* sp. BK-58. The nucleotide sequence of the *cis*-epoxysuccinate hydrolase-encoding gene is different from any of the known nucleotide sequences of *cis*-epoxysuccinate hydrolase-encoding genes. The amino acid sequence of the *cis*-epoxysuccinate hydrolase encoded by the *cis*-epoxysuccinate hydrolase-encoding gene is different from any of the amino acid sequences of known *cis*-epoxysuccinate hydrolases. The enzymatic activity of the genetically engineered bacteria containing the *cis*-epoxysuccinate hydrolase-encoding gene is higher than any of the publicly reported counterparts.

What is described above is only a summary of the technical solution of the present invention. A detailed description will be given below with reference to preferred embodiments and drawings.

## BRIEF DESCRIPTION OF THE DRAWINGS

Figure 1 shows the agarose gel electrophoresis (1%) of PCR products as discussed in example 3.

Figure 2 shows the SDS-polyacrylamide gel electrophoresis of proteins from the genetically engineered bacteria after 0.1mmol/L IPTG induction as discussed in example 4.

## DETAILED DESCRIPTION OF THE EMBODIMENTS

In order to better illustrate the technical means adopted in the present invention for achieving the object, the *cis*-epoxysuccinate hydrolase-encoding gene, the polypeptide encoded by the same and the related application thereof will be described in detail below with reference to the drawings, specific embodiments, structures, features and technical effects thereof.

Examples includes the following aspects: separating *cis*-epoxysuccinate hydrolase from *Klebsiella* sp., sequencing the N-terminal and C-terminal sequences of *cis*-epoxysuccinate hydrolase, designing degenerate primers according to amino acid sequences of N-terminal and C-terminal, cloning *cis*-epoxysuccinate hydrolase-encoding gene from *Klebsiella* sp., constructing prokaryotic expression vectors, transforming *E. coli,* culturing *E. coli* transformed with *cis*-epoxysuccinate hydrolase gene, and verifying functions of *cis*-epoxysuccinate hydrolase gene. These aspects may relate to and be further illustrated with a corresponding embodiment described as follows.

### Example 1: separation and purification of cis-epoxysuccinate hydrolase

*Klebsiella* sp. BK-58 bacteria are stored on LB slant culture medium, wherein the slant culture medium (pH 7.0) is composed of 1% peptone, 1% NaCl, 0.5% yeast extract and 1.5% agar.

### 1. Determination of cis-epoxysuccinate hydrolase activity

About 1.0 g wet cells are washed twice with normal saline and suspended in 10 mL of 1 mol/L sodium *cis*-epoxysuccinate (pH 8.0), a reaction was carried out for 1 hour at 37°C, and the content of tartaric acid in the reaction liquid is determined.

A method for determining the content of tartaric acid includes the following steps: adding successively 2.5 mL of 1% ammonium meta-vanadate solution, a defined amount of the sample and 1 mL of 1 mol/L sulfuric acid into a 25-mL volumetric flask and diluting with distilled water to scale mark, mixing thoroughly and taking a portion of the above reaction liquid to determine light absorption value at 480 nm with a spectrophotometer, and calculating the content of tartaric acid in the sample according to an established standard curve.

One unit of enzymatic activity is defined as the amount of enzyme capable of generating 1 μmol tartaric acid

by 1.0 g wet cells in 1 hour under the experimental conditions described above.

A determination method of *cis*-epoxysuccinate hydrolase activity in soluble enzyme samples includes the following steps: adding 1.0 mL of 1 mol/L sodium *cis*-epoxysuccinate to 3.9 mL of 0.1 mol/L potassium phosphate buffer solution (pH 8.0), maintaining the temperature of the mixture at 37°C for 5 minutes, adding 0.1 mL of enzyme samples to trigger a enzymatic reaction at 37°C for 20 minutes, and determining the content of tartaric acid according to the determination method described above.

**2. Separation and purification of *cis*-epoxysuccinate hydrolase**

(1) The seed broth is prepared by inoculating a loop of *Klebsiella* sp. BK-58 bacteria from slant culture medium into the LB medium and culturing on a shaker for 24 hours at 30°C. The LB medium (pH 7.0) is composed of 1% peptone, 1% NaCl and 0.5% yeast extract.
(2) *cis*-Epoxysuccinate hydrolase is expressed in the fermentation medium by inoculating the seed broth and culturing on a shaker at 30°C for 24 hours. The fermentation medium (pH 7.0) is composed of 1% peptone, 1% NaCl, 0.5% yeast extract and 1% sodium *cis*-epoxysuccinate.
(3) Cells are collected by centrifugation and suspended with 0.1 mol/L potassium phosphate buffer solution.
(4) The bacterial suspension is treated with an ultrasonic disruptor for 30 minutes and centrifuged at 8,000 rpm for 30 minutes to collect supernatant 1.
(5) Ammonium sulfate is added to the supernatant 1 to achieve 40% saturation, and supernatant 2 is collected through centrifugation at 10,000 rpm for 20 minutes.
(6) Ammonium sulfate is added to the supernatant 2 to achieve 75% saturation, and precipitate is collected through centrifugation at 10,000 rpm for 20 minutes and dissolved with 0.1 mol/L pre-cooled potassium phosphate buffer solution.
(7) Performing dialysis at 4°C for 48 hours during which dialysate is renewed for 3 or 4 times.
(8) The dialyzed enzyme liquid is passed through a DEAE-Sepharose column (4.0 cm i.d. × 50 cm) at a flow velocity of 1 mL/min. The column effluent exhibits enzymatic activity.
(9) The column effluent is concentrated with PEG 20000. The concentrated liquid is passed through a Pheny-Sepharose column (2.6 cm i.d. × 50 cm) and eluted with 0.1 mol/L potassium phosphate buffer solution (pH 8.0) at a flow velocity of 1 mL/min. Effluent with enzymatic activity is collected and concentrated with PEG 20000.
(10) The concentrated liquid is applied to a MonoQ HR5/5 column and eluted with 0.1 mol/L potassium phosphate buffer solution (pH 8.0) at a flow velocity of 1 mL/min. Effluent with enzymatic activity is collected and concentrated with PEG 20000.
(11) Liquid collected after repeating steps (8) to (10) three times is applied to SDS-polyacrylamide gel electrophoresis (PAGE), and stained with Coomassie brilliant blue R250. The purified *cis*-epoxysuccinate hydrolase is found as a single band in SDS-PAGE gel and has a molecular mass of about 30 kDa.

**Example 2: sequencing N-terminal and C-terminal of *cis*-epoxysuccinate hydrolase**

A common method is used for detecting amino acid sequences of N-terminal and C-terminal of *cis*-epoxysuccinate hydrolase, which includes the following steps: the separated and purified *cis*-epoxysuccinate hydrolase after SDS-PAGE is transferred to a PVDF film through Western blot; the film is stained with Coomassie brilliant blue; a gel slice containing *cis*-epoxysuccinate hydrolase is excised; *cis*-epoxysuccinate hydrolase is recovered; N-terminal sequence of 10 amino acids detected by a protein sequencing machine ABI PROCISETM494cLC is MKFSGASLFA (SEQ ID NO. 3); and C-terminal sequence of 10 amino acids detected by a protein sequencing machine ABI PROCISETM491cLC is VVELAGMLGA (SEQ ID NO. 4).

**Example 3: designing degenerate primers and cloning *cis*-epoxysuccinate hydrolase gene**

Based on the above-mentioned N-terminal sequence (SEQ ID NO. 3) and C-terminal sequence (SEQ ID NO. 4) of *cis*-epoxysuccinate hydrolase and the non-coding termination codon sequences (TAA/TGA/TAG), two degenerate primers are designed as follows:

Primer 1: 5'-ATGAARTTYWSNGGNGCNWSNYTNTTYGCN-3'(SEQ ID NO. 5);
Primer 2: 5'-YYANGCNCCNARCATNCCNGCNARYTCNACNAC-3'(SEQ ID NO. 6);

wherein, R: A/G, Y: C/T, W: A/T, S: G/C, V: A/G/C, N: A/G/C/T.

Steps for cloning *cis*-epoxysuccinate hydrolase gene using the degenerate primers are as follows:

(1) Culturing *Klebsiella* sp. BK-58 bacteria with LB liquid culture medium on a shaker set at 200 rpm and 30°C, collecting cells after 12 hours, and extracting genomes of *Klebsiella* sp. BK-58 according to the method described in AxyPrep Bacterial Genomic DNA MiniPrep Kit (AXYGEN).
(2) PCR amplification is performed using the genomes as templates and primers 1 and 2 as primers in a total volume of 50 μL including templates obtained from step (1) 3 μL, Taq DNA polymerase (5 M/μL) 1μL, 10 × PCR buffer 5 μL, $MgCl_2$ (25 mmol/L) 3 μL, primer 1 (10 μmol/μL) 1 μL, primer 2 (10 μmol/μL) 1 μL, dNTP (100 mmol/L) 1 μL, and $H_2O$ 35 μL.
(3) The above-mentioned 50 μL PCR reagents are subject to the following procedure:
preheating at 94°C for 5 minutes; maintaining the reaction temperature at 94°C for 50 seconds, 50°C for 30 seconds and 72°C for 1 minute; repeating the second step for 30 cycles; and then maintaining at 72°C for 10 minutes.
(4) The PCR products are applied to 1% agarose gel electrophoresis. The result thereof is shown in figure 1. A clear band is visible in lane 1 between 750 bp and 1000 bp, and no corresponding band in lane 2 (control) is observed. PCR products in the clear band in lane 1 is recovered and joined to vector pUCm-T, and then transformed into *E. coli* DH5 α. Colonies are picked up for sequencing validation. According to the sequencing result shown in SEQ ID NO. 1, the *cis*-epoxysuccinate hydrolase has 825 bp of length with ATG as the start codon and TAA as the termination codon. The nucleotide sequence shown in SEQ ID NO. 1 is translated to an amino acid sequence shown in SEQ ID NO. 2 (that is the amino acid sequence of *cis*-epoxysuccinate hydrolase) through a software BioXM. The similarity between all the sequences available in the online databases and the encoding gene sequence and amino acid sequence of the *cis*-epoxysuccinate hydrolase obtained is found no more than 50%, which suggests that the obtained *cis*-epoxysuccinate hydrolase-encoding gene is a novel gene.

**Example 4: construction of prokaryotic expression vector for *cis*-epoxysuccinate hydrolase gene from *Klebsiella* sp. and expression of the vector in *E. coli***

Forward primer 3 containing the recognition site of restriction enzyme Nde I and reverse primer 4 containing the recognition site of restriction enzyme BamH I are designed according to the sequence shown in SEQ ID NO. 1. Sequences of primers 3 and 4 are:

Primer 3: 5'-CATATGAAATTTTCTGGCGCCTCT (SEQ ID NO. 7);
Primer 4: 5'-GGATCCTTAGGCGCCCAGCAT (SEQ ID NO. 8).

PCR is carried out using genomes of *Klebsiella* sp. as template, and primers 3 and 4 as primers in a total volume of 50 μL including template obtained from step (1) in example 3 3 μL, Taq DNA polymerase (5 M/μL) 1 μL, 10 × PCR buffer 5 μL, $MgCl_2$ (25 mmol/L) 3 μL, primer 3 (10 μmol/μL) 1 μL, primer 4 (10 μmol/μL) 1 μL, dNTP (100 mmol/L) 1 μL, and $H_2O$ 35 μL.
The above-mentioned 50 μL PCR reagents are subject to the following procedure:
preheating at 94°C for 5 minutes; maintaining the reaction temperature at 94°C for 50 seconds, 44°C for 30 seconds and 72°C for 1 minute; repeating step two for 30 cycles; and then maintaining at 72°C for 10 minutes.
The PCR products are recovered and joined with vector pUCm-T, and transformed into *E. coli* DH5 α. Colonies are picked up for sequencing validation. The sequencing result is aligned to the *cis*-epoxysuccinate hydrolase-encoding gene shown in SEQ ID NO. 1. The result shows that *cis*-epoxysuccinate hydrolase-encoding genes containing the recognition sites of restriction enzymes Nde I and BamH I are successfully inserted into pUCm-T vector. Vector pUCm-T containing *cis*-epoxysuccinate hydrolase-encoding gene and expression vector pET-22b(+) are digested with both restriction enzymes Nde I and BamH I, respectively. The cleaved *cis*-epoxysuccinate hydrolase-encoding gene are joined with the digested vector pET-22b(+) using T4 DNA ligase, which are then transformed into *E. coli* BL21 (DE3). The colonies are picked up for sequencing. The complete match between the sequencing result and the *cis*-epoxysuccinate hydrolase gene implies that pET-22b(+) recombinant vector containing *cis*-epoxysuccinate hydrolase gene and the genetically engineered bacteria have been successfully constructed.
The genetically engineered bacteria are applied to SDS-PAGE after 0.01 mmol/L IPTG induction, and the result thereof is shown in figure 2. There is a dark band in lane 3 (sample of cell lysate of genetically engineered bacteria after IPTG induction) between 25 kDa and 35 kDa, and the molecular mass of protein shown by the band conforms to the molecular mass calculated according to the amino acid sequence of *cis*-epoxysuccinate hydrolase. There is no corresponding protein band in lane 2 (used as a control) which represents *E. coli* BL21 containing vector pET-22b(+) without any foreign nucleotide fragment inserted, indicating the recombinant protein encoded by *cis*-epoxysuccinate hydrolase gene can be efficiently expressed. According to the method for determining *cis*-epoxysuccinate hydrolase activity as described in example 1, the control bacteria shows no *cis*-epoxysuccinate hydrolase activity, but the genetically engineered bacteria containing *cis*-epoxysuccinate hydrolase gene shows an enzymatic activity of 35,600 U/g.

**Example 5: preparing L(+)-tartaric acid using the genetically engineered bacteria**

In this example, infrared spectrum of a sample is taken on a Nicolet-Nexus 670 Fourier transform infrared spectrometer; H and C nuclear magnetic resonance spectra of a sample are observed by a Bruker Avance DMX500 nuclear magnetic resonance spectrometer; mass spectrum of a sample is recorded by a Bruker Esquire 3000 plus mass spectrometer; and optical rotation of a sample is detected by a WZZ-2B polarimeter.

100 mL of seed broth of the genetically engineered bacteria after culturing in LB medium on a shaker for 12 hours at 37°C as described in example 4, is inoculated into 1 L of LB medium and cultured on a shaker (200 rpm) at 37°C for 10 hours, after which 10 g of disodium *cis*-epoxysuccinate is added and the culture continues for another 12 hours. An excess of $CaCl_2$ aqueous solution is added into the fermentation broth for precipitating the product. The precipitate is washed with water to obtain 14 g calcium tartrate. The calcium tartrate is acidified with sulfuric acid, and the crude tartaric acid solution is refined by anion and cation-exchange chromatography techniques. After concentration, crystallization and drying, 6.7 g solid product is obtained. The solid product is identified to be tartaric acid through infrared spectrum, ultraviolet spectrum, nuclear magnetic resonance spectrum, and mass spectrum inspection. The specific rotation $([\alpha]_D^{20})$ of the solid product is +12.1°, which indicates that the solid product is L(+)-tartaric acid with a purity of 99.9%.

The disodium *cis*-epoxysuccinate in this example can be alternated by *cis*-epoxysuccinic acid or salts formed by *cis*-epoxysuccinic acid and various cations, wherein such cations may be ammonium ion, potassium ion, magnesium ion, and/or calcium ion, etc.

**Example 6: constructing an expression vector of encoding gene of polypeptide having *cis*-epoxysuccinate hydrolase activity and an amino acid sequence as shown in SEQ ID NO. 2 with one or more amino acids substituted, deleted or added and expressing the vector in *E. coli***

**1. Randomly adding a nucleotide sequence to the 5'-terminal of the *cis*-epoxysuccinate hydrolase gene**

A forward primer 5 containing a restriction enzyme Nde I recognition site and a randomly added nucleotide sequence according to the sequence shown in SEQ ID NO. 1 is designed as follows:
Primer 5: 5'-CATATGGACCGTCGTCAGTTCATGAAATTTTCTGGCGCCTCT (SEQ ID NO. 9).

PCR is carried out using genomes of *Klebsiella* sp. BK-58 as templates, and primers 3 and 4 as primers in a total volume of 50 μL including: templates obtained from step (1) in example 3 3 μL, Taq DNA polymerase (5 M/μL) 1 μL, 10×PCR buffer 5 μL, $MgCl_2$ (25 mmol/L) 3 μL, primer 5 (10 μmol/μL) 1 μL, primer 4 (10 μmol/μL) 1 μL, dNTP (100 mmol/L) 1 μL, and $H_2O$ 35 μL.

The above-mentioned 50 μL PCR reagents are subject to the following PCR procedure:
preheating at 94°C for 5 minutes; maintaining the reaction temperature at 94°C for 50 seconds, 44°C for 30 seconds and 72°C for 1 minute; repeating step 2 for 30 cycles; and maintaining at 72°C for 10 minutes.

The PCR products are recovered and joined with vector pUCm-T, and transformed into *E. coli* DH5 α. Colonies are picked up for sequencing validation. The sequencing result is shown in SEQ ID NO. 10. Aligning the sequencing result to *cis*-epoxysuccinate hydrolase-encoding gene (as shown in SEQ ID NO. 1), it is found that the sequence as shown in SEQ ID NO. 10 is equivalent to the sequence as shown in SEQ ID NO. 1 with a sequence of ATGGACCGTCGTCAGTTC (SEQ ID NO. 11) added to the 5'-terminal thereof. The amino acid sequence encoded by the sequence shown in SEQ ID NO. 10 is shown in SEQ ID NO. 12, which is equivalent to the amino acid sequence as shown in SEQ ID NO. 2 with a sequence of six amino acids (SEQ ID NO. 13) added at the N-terminal thereof. Vector pUCm-T containing sequences as shown in SEQ ID NO. 10 and expression vector pET-22b(+) are digested with both restriction enzymes Nde I and BamH I, respectively. The cleaved sequence as shown in SEQ ID NO. 10 are joined with the digested vector pET-22b(+) using T4 DNA ligase. The joined products are transformed into *E. coli* BL21 (DE3) to construct a recombinant vector pET-22b(+) containing the sequence as showed in SEQ ID NO. 10 and genetically engineered bacteria containing the recombinant vector pET-22b(+).

The genetically engineered bacteria are cultured in LB culture medium containing 100 μg/mL ampicillin on a shaker at 37°C overnight. *E. coli* BL21 containing vector pET-22b(+) without any foreign nucleotide fragment inserted is used as a control. According to the determination method of *cis*-epoxysuccinate hydrolase activity described in example 1, the bacteria as a control shows no *cis*-epoxysuccinate hydrolase activity, but the genetically engineered bacteria described above show an enzymatic activity of 38,800 U/g.

Using the genetically engineered bacteria described in this example, L(+)-tartaric acid with a purity of 99.9% is obtained through the preparation method of L(+)-tartaric acid in example 5.

**2. Randomly adding a nucleotide sequence to the 3'-terminal of the *cis*-epoxysuccinate hydrolase gene**

A reverse primer 6 containing a restriction enzyme BamH I recognition site and a randomly added nucleotide sequence according to the sequence shown in SEQ ID NO. 1 is designed as follows:
Primer 6: 5'-GGATCCTTACTTGTTCGGCGTCTGTTTGGCGCCCAGCAT (SEQ ID NO. 14).

PCR is carried out using genomes of *Klebsiella* sp. BK-58 as templates, and primer 3 in example 4 and primer 6 as primers in a total volume of 50 μL including: templates obtained from step (1) in example 33 μL, Taq DNA polymerase (5 M/μL) 1 μL, 10 × PCR buffer 5 μL, $MgCl_2$ (25 mmol/L) 3 μL, primer 6 (10 μmol/μL) 1 μL, primer 3 (10 μmol/μL) 1 μL, dNTP (100 mmol/L) 1 μL, and $H_2O$ 35 μL.

The above-mentioned 50 μL PCR reagents are subject to the following PCR procedure:
preheating at 94°C for 5 minutes; maintaining the reaction temperature at 94°C for 50 seconds, 44°C for 30 seconds and 72°C for 1 minute; repeating step two for 30 cycles; and then maintaining at 72°C for 10 minutes.

The PCR products are recovered and joined with vector pUCm-T, and transformed into *E. coli* DH5 α. Colonies are picked up for sequencing validation. Aligning the sequencing result shown in SEQ ID NO. 15 to *cis*-epoxysuccinate hydrolase-encoding gene (as shown in SEQ ID NO. 1), it was observed that the sequence as shown in SEQ ID NO. 15 is equivalent to the sequence of *cis*-epoxysuccinate hydrolase gene with its stop codon TAA mutated into AAA and its 3'-terminal added by a sequence of CAGACGCCGAACAAGTAA (SEQ ID NO. 16). The amino acid sequence encoded by the sequence as shown in SEQ ID NO. 15 is shown in SEQ ID NO. 17, which is equivalent to the amino sequence as shown in SEQ ID NO. 2 with its C-terminal added by a sequence of six amino acids (SEQ ID NO. 18). Vector pUCm-T containing sequence as shown in SEQ ID NO. 15 and expression vector pET-22b(+) are digested with both restriction enzymes Nde I and BamH I, respectively. The cleaved sequence as shown in SEQ ID NO. 15 is joined with the digested vector pET-22b(+) using T4 DNA ligase, which is then transformed into *E. coli* BL21 (DE3) to construct a recombinant vector pET-22b(+) containing the sequence as showed in SEQ ID NO. 15 and genetically engineered bacteria containing the recombinant vector pET-22b(+).

The genetically engineered bacteria are cultured in LB culture medium containing 100 μg/mL ampicillin on a shaker at 37°C overnight. *E. coli* BL21 containing vector pET-22b(+) without any foreign nucleotides insertion fragments are used as a control. According to method for determining *cis*-epoxysuccinate hydrolase activity as described in example 1, the control bacteria show no *cis*-epoxysuccinate hydrolase activity, but the genetically engineered bacteria described above show an enzymatic activity of 35,800 U/g.

Using the genetically engineered bacteria described in this example, L(+)-tartaric acid with a purity of 99.9% is obtained through the preparation method of L(+)-tartaric acid in example 5.

**3. Randomly adding a nucleotide sequences to both the 5'-terminal and 3'-terminal of the cis-epoxysuccinate hydrolase gene**

PCR is carried out using genomes of *Klebsiella* sp. BK-58 as templates, and primers 5 and 6 as primers in a total volume of 50 μL including: templates obtained from step (1) in example 3 3 μL, Taq DNA polymerase (5 M/μL) 1 μL, 10×PCR buffer 5 μL, $MgCl_2$ (25 mmol/L) 3 μL, primer 5 (10 μmol/μL) 1 μL, primer 6 (10 μmol/μL) 1 μL, dNTP (100 mmol/L) 1 μL, and $H_2O$ 35 μL.

The above-mentioned 50 μL PCR reagents are subject to the following PCR procedure:
preheating at 94°C for 5 minutes; maintaining the reaction temperature at 94°C for 50 seconds, 54°C for 30 seconds and 72°C for 1 minute; repeating step two for 30 cycles; and then maintaining the reaction temperature at 72°C for 10 minutes.

The PCR products are recovered and joined with vector pUCm-T, and transformed into *E. coli* DH5 α. Colonies were picked up for sequencing validation. By aligning the sequencing result shown in SEQ ID NO. 19 to the *cis*-epoxysuccinate hydrolase-encoding gene (as shown in SEQ ID NO. 1), it is found that the sequence as shown in SEQ ID NO. 19 is equivalent to the sequence as shown in SEQ ID NO. 1 with its 5'-terminal added by a sequence ATGGACCGTCGTCAGTTC (SEQ ID NO. 11), stop codon TAA of *cis*-epoxysuccinate hydrolase gene mutated into AAA and its 3'-terminal added by a sequence CAGACGCCGAACAAGTAA (SEQ ID NO. 16). The amino acid sequence encoded by the sequence as shown in SEQ ID NO. 19 is shown in SEQ ID NO. 20, which is equivalent to the amino sequence as shown in SEQ ID NO. 2 with its N-terminal added by a sequence of six amino acids (SEQ ID NO. 13) and its C-terminal added by a sequence of six amino acids (SEQ ID NO. 18). Vector pUCm-T containing sequence as shown in SEQ ID NO. 19 and expression vector pET-22b(+) are digested with both restriction enzymes Nde I and BamH I, respectively. The cleaved sequences as shown in SEQ ID NO. 19 are joined with the digested vector pET-22b(+) using T4 DNA ligase, which is then transformed into *E. coli* BL21 (DE3) to construct a recombinant vector pET-22b(+) containing the sequence as shown in SEQ ID NO. 19 and genetically engineered bacteria containing the recombinant vector pET-22b(+).

The genetically engineered bacteria are cultured in LB culture medium containing 100 μg/mL ampicillin on a shaker at 37°C overnight. *E. coli* BL21 containing vector pET-22b(+) without any foreign nucleotide fragment inserted is used as a control. According to the method for determining *cis*-epoxysuccinate hydrolase activity described in example 1, the control bacteria show no *cis*-epoxysuccinate hydrolase activity, but the genetically engineered bacteria described above show an enzymatic activity of 36,200 U/g.

Using the genetically engineered bacteria described in this example, L(+)-tartaric acid with a purity of 99.9% is obtained through the preparation method of L(+)-tartaric acid in example 5.

**4. Substituting a nucleotide sequence in the *cis*-epoxysuccinate hydrolase gene**

Primers 7 and 8 are designed according to the sequence shown in SEQ ID NO. 1, and their sequences are as follows:

Primer 7: 5'-AGTGATCAAGGGGGAAAAACCGTGGACCACGACAG (SEQ ID NO. 21);
Primer 8: 5'-CTGTCGTGGTCCACGGTTTTTCCCCCTTGATCACT (SEQ ID NO. 22).

PCR is carried out using recombinant vector pET-22b(+) containing *cis*-epoxysuccinate hydrolase genes in example 4 as templates, and primers 7 and 8 as primers in a total volume of 50 μL including: templates 3 μL, SuperLA Taq DNA polymerase (5 M/μL) 1 μL, 10×PCR buffer 5 μL, primer 7 (10 μmol/μL) 2 μL, primer 8 (10 μmol/μL) 2 μL, dNTP (100 mmol/L) 1 μL, and $H_2O$ 36 μL.

The above-mentioned 50 μL PCR reagents are subject to the following PCR procedure:
preheating at 94°C for 5 minutes; maintaining the reaction temperature at 94°C for 50 seconds, 56°C for 30 seconds and 72°C for 7 minutes; repeating step two for 25 cycles; and then maintaining at 72°C for 10 minutes.

The PCR products are transformed into *E. coli* BL21(DE3) to construct genetically engineered bacteria. Colonies are picked up for sequencing. By aligning the sequencing result (SEQ ID NO. 23) to the *cis*-epoxysuccinate hydrolase gene (as shown in SEQ ID NO. 1), it is found that the sequence as shown in SEQ ID NO. 23 is equivalent to the sequence as shown in SEQ ID NO. 1 with its $299^{th}$ and $300^{th}$ nucleotides GG substituted by AA. The amino acid sequence shown in SEQ ID NO. 24 encoded by the nucleotide sequence as shown in SEQ ID NO. 23 is equivalent to the amino acid sequence as shown in SEQ ID NO. 2 with its $100^{th}$ amino acid arginine substituted by lysine.

The genetically engineered bacteria are cultured in LB culture medium containing 100 μg/mL ampicillin on a shaker at 37°C overnight. *E. coli* BL21 containing vector pET-22b(+) without any foreign nucleotide fragment inserted is used as a control. According to method for determining *cis*-epoxysuccinate hydrolase activity described in example 1, the bacteria as a control shows no *cis*-epoxysuccinate hydrolase activity, but the genetically engineered bacteria described above show an enzymatic activity of 32,400 U/g.

Using the genetically engineered bacteria described in this example, L(+)-tartaric acid with a purity of 99.9% is obtained through the preparation method of L(+)-tartaric acid in example 5.

**5. Deleting a nucleotide sequence from the 5'terminal of the *cis*-epoxysuccinate hydrolase gene**

Forward primer 9 containing a restriction enzyme Nde I recognition site is designed according to the sequence shown in SEQ ID NO. 1, and the sequence of primer 9 is as follows:
Primer 9: 5'-CATATG AACCAGATGGCCTTA (SEQ ID NO. 25).

PCR is carried out using genomes of *Klebsiella* sp. BK-58 as templates, and primer 4 in example 4 and primer 9 as primers in a total volume of 50 μL including: templates obtained from step (1) in example 3 3 μL, Taq DNA polymerase (5 M/μL) 1 μL, 10×PCR buffer 5 μL, $MgCl_2$ (25 mmol/L) 3 μL, primer 9 (10 μmol/μL) 1 μL, primer 4 (10 μmol/μL) 1 μL, dNTP (100 mmol/L) 1 μL, and $H_2O$ 35 μL.

The above-mentioned 50 μL PCR reagents are subject to the following PCR procedure:
preheating at 94°C for 5 minutes; maintaining the reaction temperature at 94°C for 50 seconds, 44°C for 30seconds and 72°C for 1 minute; repeating step 2 for 30 cycles; and then maintaining at 72°C for 10 minutes.

The PCR products are recovered and joined with vector pUCm-T, and transformed into *E. coli* DH5 α. Colonies are picked up for sequencing validation. By aligning the sequencing result shown in SEQ ID NO. 26 to the *cis*-epoxysuccinate hydrolase-encoding gene (as shown in SEQ ID NO. 1), it is found that the sequence as shown in SEQ ID NO. 26 is equivalent to the sequence as shown in SEQ ID NO. 1 with a sequence (AACCAGATGGCCTTAAAGCCCTGTTCTTTGACGTCCAGGGAACACTTGTC GATTTTTATTCGACAATCACCCGAGAGGGCGAAGCCTTCTCAGCTGTTC) deleted from5'-terminal thereof. The amino acid sequence shown in SEQ ID NO. 27 encoded by the sequence as shown in SEQ ID NO. 26 is equivalent to the amino sequence as shown in SEQ ID NO. 2 with a sequence of 33 amino acids deleted from the N-terminal thereof. Vector pUCm-T containing sequence as shown in SEQ ID NO. 26 and expression vector pET-22b(+) are digested with restriction enzymes Nde I and BamH I, respectively. The cleaved sequence as shown in SEQ ID NO. 26 are joined with the digested vector pET-22b(+) using T4 DNA ligase, which is transformed into *E. coli* BL21 (DE3) to construct a recombinant vector pET-22b(+)containing the sequence as showed in SEQ ID NO. 26 and genetically engineered bacteria containing the recombinant vector pET-22b(+).

The genetically engineered bacteria are cultured in LB culture medium containing 100 μg/mL ampicillin on a shaker at 37°C overnight. *E. coli* BL21 containing vector pET-22b(+) without any foreign nucleotide fragment inserted is used as a control. According to the method for determining *cis*-epoxysuccinate hydrolase activity described in example 1, the bacteria as a control shows no *cis*-epoxysuccinate hydrolase activity, but the genetically engineered bacteria described above show an enzymatic activity of 33,600 U/g.

Using the genetically engineered bacteria described in this example, L(+)-tartaric acid with a purity of 99.9% is obtained through the preparation method of L(+)-tartaric acid in example 5.

Sequence listing

<110>Hangzhou Bioking Biochemical Engineering Co., Ltd.
<120>CIS-EPOXYSUCCINATE HYDROLASE-ENCODING GENE, POLYPEPTIDE ENCODED BY THE GENE, AND RELATED APPLICATION THEREOF
<160>27
<170>PatentIn version 3.1
<210>1
<211>825
<212>DNA
<213>Klebsiella sp.
<400>1

```
atgaaatttctggcgcctctctctttgcagctgtgtctggcgcctctctctttgcagct    60
gtgagtagctcaaacacttttgccgatgcgtctttgatccggaagggcggccaaccagat   120
ggccttaaagccctgttctttgacgtccagggaacacttgtcgattttattcgacaatc    180
acccgagagggcgaagccttctcagctgttcgaggcttccaagctgattggacaacggtc   240
accgagcaatggcgagccgagtatcgcagtcgtttggaccaagtgatcaaggggaaagg    300
ccgtggaccacgacagacaggatctatcgcgaggcgctggatggaatactcgccaaccat   360
ccttggggagcgtccctcaattctgcagatcgtgacgaactaaatagcctgtggtccaaa   420
ctgattccgtgggatgacactgctccgggtcttgcgagactgaggtcgaaatacataact   480
tcgactttgtcgaatggcagcatggcatctgtccttcgaatttcgaagcttggcgctctt   540
ccatttgatgcgatccttactgcggagttggtgcggtcatcaaagcctgacccaaaggtg   600
tatcaacttgcactggactcagttggcattgaggcgcatcaagctatgatggtagcttgc   660
cataaatacgaccttcaagcagcaaagcggcttggttttaaagttgcctttattgcgcga   720
cccttcgagtttggaccgaacaaaaaagttgacacaaaaccagagcagtattttgattat   780
tatgcaaattctgttgttgagctagctggcatgctgggcgcctaa                  825
```

<210>2
<211> 274
<212>PRT
<213>Klebsiella sp.
<400>2

```
MKFSGASLFAAVSGASLFAAVSSSNTFADASLIRKGGQPDGLKALFFDVQGTLVDFYSTI    60
TREGEAFSAVRGFQADWTTVTEQWRAEYRSRLDQVIKGERPWTTTDRIYREALDGILANH   120
PWGASLNSADRDELNSLWSKLIPWDDTAPGLARLRSKYITSTLSNGSMASVLRISKLGAL   180
PFDAILTAELVRSSKPDPKVYQLALDSVGIEAHQAMMVACHKYDLQAAKRLGFKVAFIAR   240
PFEFGPNKKVDTKPEQYFDYYANSVVELAGMLGA                             274
```

<210>3
<211>10
<212>Polypeptide
<213>Klebsiella sp.
<400>3

MKFSGASLFA
<210>4 <211>10 <212>Polypeptide <213>Klebsiella sp. <400>4
VVELAGMLGA
<210>5
<211>30
<212>DNA
<213>Artificial sequence
<400>5
ATGAARTTYWSNGGNGCNWSNYTNTTYGCN
<210>6
<211>33
<212>DNA
<213>Artificial sequence
<400>6
YYANGCNCCNARCATNCCNGCNARYTCNACNAC
<210>7
<211> 24
<212> DNA
<213>Artificial sequence
<400>7
CATATGAAATTTTCTGGCGCCTCT
<210>8
<211>21
<212>DNA
<213>Artificial sequence
<400>8
GGATCCTTAGGCGCCCAGCAT
<210>9
<211>42
<212>DNA
<213>Artificial sequence
<400>9
CATATGGACCGTCGTCAGTTCATGAAATTTTCTGGCGCCTCT
<210>10
<211> 843
<212>DNA
<213>Artificial sequence
<400>10

```
atggaccgtcgtcagttcatgaaattttctggcgcctctctctttgcagctgtgtctggc     60
gcctctctctttgcagctgtgagtagctcaaacacttttgccgatgcgtctttgatccgg     120
aagggcggccaaccagatggccttaaagccctgttctttgacgtccagggaacacttgtc   180
gatttttattcgacaatcacccgagagggcgaagccttctcagctgttcgaggcttccaa     240
gctgattggacaacggtcaccgagcaatggcgagccgagtatcgcagtcgtttggaccaa   300
gtgatcaaggggaaaggccgtggaccacgacagacaggatctatcgcgaggcgctggat  360
ggaatactcgccaaccatccttggggagcgtccctcaattctgcagatcgtgacgaacta    420
aatagcctgtggtccaaactgattccgtgggatgacactgctccgggtcttgcgagactg    480
aggtcgaaatacataacttcgactttgtcgaatggcagcatggcatctgtccttcgaatt     540
tcgaagcttggcgctcttccatttgatgcgatccttactgcggagttggtgcggtcatca     600
aagcctgacccaaaggtgtatcaacttgcactggactcagttggcattgaggcgcatcaa   660
gctatgatggtagcttgccataaatacgaccttcaagcagcaaagcggcttggttttaaa    720
gttgcctttattgcgcgacccttcgagtttggaccgaacaaaaaagttgacacaaaacca   780
gagcagtattttgattattatgcaaattctgttgttgagctagctggcatgctgggcgcc     840
taa                                                        843
```

<210> 11
<211> 18
<212> DNA

<213>Artificial sequence
<400>11
ATGGACCGTCGTCAGTTC
<210>12
<211>280
<212>PRT
<213>Artificial sequence
<400>12

MDRRQFMKFSGASLFAAVSGASLFAAVSSSNTFADASLIRKGGQPDGLKALFFDVQGTLV 60
DFYSTITREGEAFSAVRGFQADWTTVTEQWRAEYRSRLDQVIKGERPWTTTDRIYREALD 120
GILANHPWGASLNSADRDELNSLWSKLIPWDDTAPGLARLRSKYITSTLSNGSMASVLRI 180
SKLGALPFDAILTAELVRSSKPDPKVYQLALDSVGIEAHQAMMVACHKYDLQAAKRLGFK 240
VAFIARPFEFGPNKKVDTKPEQYFDYYANSVVELAGMLGA 280

<210>13
<211>6
<212>Polypeptide
<213>Artificial sequence
<400>13
MDRRQF
<210>14
<211>39
<212> DNA
<213>Artificial sequence
<400>14
GGATCCTTACTTGTTCGGCGTCTGTTTGGCGCCCAGCAT
<210>15
<211>843
<212>DNA
<213>Artificial sequence
<400>15

atgaaattttctggcgcctctctctttgcagctgtgtctggcgcctctctctttgcagct 60
gtgagtagctcaaacacttttgccgatgcgtctttgatccggaagggcggccaaccagat 120
ggccttaaagccctgttctttgacgtccagggaacacttgtcgatttttattcgacaatc 180
acccgagagggcgaagccttctcagctgttcgaggcttccaagctgattggacaacggtc 240
accgagcaatggcgagccgagtatcgcagtcgtttggaccaagtgatcaaggggggaaagg 300
ccgtggaccacgacagacaggatctatcgcgaggcgctggatggaatactcgccaaccat 360
ccttggggagcgtccctcaattctgcagatcgtgacgaactaaatagcctgtggtccaaa 420
ctgattccgtgggatgacactgctccgggtcttgcgagactgaggtcgaaatacataact 480
tcgactttgtcgaatggcagcatggcatctgtccttcgaatttcgaagcttggcgctctt 540
ccatttgatgcgatccttactgcggagttggtgcggtcatcaaagcctgacccaaaggtg 600
tatcaacttgcactggactcagttggcattgaggcgcatcaagctatgatggtagcttgc 660
cataaatacgaccttcaagcagcaaagcggcttggttttaaagttgcctttattgcgcga 720
cccttcgagtttggaccgaacaaaaaagttgacacaaaaccagagcagtattttgattat 780
tatgcaaattctgttgttgagctagctggcatgctgggcgccaaacagacgccgaacaag 840
taa 843

<210>16
<211> 18
<212> DNA
<213>Artificial sequence
<400>16
cagacgccgaacaagtaa
<210>17
<211>280
<212> PRT

<213>Artificial sequence
<400>17

```
MKFSGASLFAAVSGASLFAAVSSSNTFADASLIRKGGQPDGLKALFFDVQGTLVDFYSTI     60
TREGEAFSAVRGFQADWTTVTEQWRAEYRSRLDQVIKGERPWTTTDRIYREALDGILANH 120
PWGASLNSADRDELNSLWSKLIPWDDTAPGLARLRSKYITSTLSNGSMASVLRISKLGAL  180
PFDAILTAELVRSSKPDPKVYQLALDSVGIEAHQAMMVACHKYDLQAAKRLGFKVAFIAR  240
PFEFGPNKKVDTKPEQYFDYYANSVVELAGMLGAKQTPNK                        280
```

<210> 18
<211>6
<212>Polypeptide
<213>Artificial sequence
<400>18
KQTPNK
<210>19
<211>861
<212>DNA
<213>Artificial sequence
<400>19

```
atggaccgtcgtcagttcatgaaattttctggcgcctctctctttgcagctgtgtctggc   60
gcctctctctttgcagctgtgagtagctcaaacacttttgccgatgcgtctttgatccgg   120
aagggcggccaaccagatggccttaaagccctgttctttgacgtccagggaacacttgtc   180
gattttattcgacaatcacccgagagggcgaagccttctcagctgttcgaggcttccaa   240
gctgattggacaacggtcaccgagcaatggcgagccgagtatcgcagtcgtttggaccaa   300
gtgatcaagggggaaaggccgtggaccacgacagacaggatctatcgcgaggcgctggat   360
ggaatactcgccaaccatccttggggagcgtccctcaattctgcagatcgtgacgaacta   420
aatagcctgtggtccaaactgattccgtgggatgacactgctccgggtcttgcgagactg   480
aggtcgaaatacataacttcgactttgtcgaatggcagcatggcatctgtccttcgaatt   540
tcgaagcttggcgctcttccatttgatgcgatccttactgcggagttggtgcggtcatca   600
aagcctgacccaaaggtgtatcaacttgcactggactcagttggcattgaggcgcatcaa   660
gctatgatggtagcttgccataaatacgaccttcaagcagcaaagcggcttggttttaaa   720
gttgcctttattgcgcgacccttcgagtttggaccgaacaaaaaagttgacacaaaacca   780
gagcagtattttgattattatgcaaattctgttgttgagctagctggcatgctgggcgcc   840
aaacagacgccgaacaagtaa                                     861
```

<210>20
<211>286
<212>PRT
<213>Artificial sequence
<400>20

```
MDRRQFMKFSGASLFAAVSGASLFAAVSSSNTFADASLIRKGGQPDGLKALFFDVQGTLV   60
DFYSTITREGEAFSAVRGFQADWTTVTEQWRAEYRSRLDQVIKGERPWTTTDRIYREALD 120
GILANHPWGASLNSADRDELNSLWSKLIPWDDTAPGLARLRSKYITSTLSNGSMASVLRI    180
SKLGALPFDAILTAELVRSSKPDPKVYQLALDSVGIEAHQAMMVACHKYDLQAAKRLGFK  240
VAFIARPFEFGPNKKVDTKPEQYFDYYANSVVELAGMLGAKQTPNK                  286
```

<210> 21
<211>35
<212> DNA
<213>Artificial sequence
<400>21
AGTGATCAAGGGGGAAAAACCGTGGACCACGACAG
<210>22
<211>35
<212>DNA

<213>Artificial sequence
<400>22
CTGTCGTGGTCCACGGTTTTTCCCCCTTGATCACT
<210>23
<211>825
<212>DNA
<213>Artificial sequence
<400>23

```
atgaaattttctggcgcctctctctttgcagctgtgtctggcgcctctctctttgcagct    60
gtgagtagctcaaacacttttgccgatgcgtctttgatccggaagggcggccaaccagat   120
ggccttaaagccctgttctttgacgtccagggaacacttgtcgattttattcgacaatc    180
acccgagagggcgaagccttctcagctgttcgaggcttccaagctgattggacaacggtc   240
accgagcaatggcgagccgagtatcgcagtcgtttggaccaagtgatcaaggggaaaaa   300
ccgtggaccacgacagacaggatctatcgcgaggcgctggatggaatactcgccaaccat   360
ccttggggagcgtccctcaattctgcagatcgtgacgaactaaatagcctgtggtccaaa   420
ctgattccgtgggatgacactgctccgggtcttgcgagactgaggtcgaaatacataact   480
tcgactttgtcgaatggcagcatggcatctgtccttcgaatttcgaagcttggcgctctt   540
ccatttgatgcgatccttactgcggagttggtgcggtcatcaaagcctgacccaaaggtg   600
tatcaacttgcactggactcagttggcattgaggcgcatcaagctatgatggtagcttgc   660
cataaatacgaccttcaagcagcaaagcggcttggttttaaagttgcctttattgcgcga   720
cccttcgagtttggaccgaacaaaaaagttgacacaaaaccagagcagtattttgattat   780
tatgcaaattctgttgttgagctagctggcatgctgggcgcctaa                  825
```

<210>24
<211>274
<212>PRT
<213>Artificial sequence
<400>24

```
MKFSGASLFAAVSGASLFAAVSSSNTFADASLIRKGGQPDGLKALFFDVQGTLVDFYSTI    60
TREGEAFSAVRGFQADWTTVTEQWRAEYRSRLDQVIKGEKPWTTTDRIYREALDGILANH   120
PWGASLNSADRDELNSLWSKLIPWDDTAPGLARLRSKYITSTLSNGSMASVLRISKLGAL   180
PFDAILTAELVRSSKPDPKVYQLALDSVGIEAHQAMMVACHKYDLQAAKRLGFKVAFIAR   240
PFEFGPNKKVDTKPEQYFDYYANSVVELAGMLGA                             274
```

<210>25
<211>21
<212>DNA
<213>Artificial sequence
<400>25
CATATGAACCAGATGGCCTTA
<210>26
<211>726
<212>DNA
<213>Artificial sequence
<400>26

```
atgaagggcggccaaccagatggccttaaagccctgttctttgacgtccagggaacactt   60
gtcgatttttattcgacaatcacccgagagggcgaagccttctcagctgttcgaggcttc   120
caagctgattggacaacggtcaccgagcaatggcgagccgagtatcgcagtcgtttggac   180
caagtgatcaaggggggaaaggccgtggaccacgacagacaggatctatcgcgaggcgctg   240
gatggaatactcgccaaccatccttggggagcgtccctcaattctgcagatcgtgacgaa   300
ctaaatagcctgtggtccaaactgattccgtgggatgacactgctccgggtcttgcgaga   360
ctgaggtcgaaatacataacttcgactttgtcgaatggcagcatggcatctgtccttcga   420
atttcgaagcttggcgctcttccatttgatgcgatccttactgcggagttggtgcggtca   480
tcaaagcctgacccaaaggtgtatcaacttgcactggactcagttggcattgaggcgcat   540
caagctatgatggtagcttgccataaatacgaccttcaagcagcaaagcggcttggtttt   600
aaagttgcctttattgcgcgacccttcgagtttggaccgaacaaaaaagttgacacaaaa   660
ccagagcagtattttgattattatgcaaattctgttgttgagctagctggcatgctgggc   720
gcctaa                                                      726
```

<210>27
<211>241
<212>PRT
<213>Artificial sequence
<400>27

```
MKGGQPDGLKALFFDVQGTLVDFYSTITREGEAFSAVRGFQADWTTVTEQWRAEYRSRLD   60
QVIKGEKPWTTTDRIYREALDGILANHPWGASLNSADRDELNSLWSKLIPWDDTAPGLAR   120
LRSKYITSTLSNGSMASVLRISKLGALPFDAILTAELVRSSKPDPKVYQLALDSVGIEAH   180
QAMMVACHKYDLQAAKRLGFKVAFIARPFEFGPNKKVDTKPEQYFDYYANSVVELAGMLG   240
A
241
```

## Claims

1. A *cis*-epoxysuccinate hydrolase-encoding gene, **characterized in that**: the *cis*-epoxysuccinate hydrolase-encoding gene includes a segment having a nucleotide sequence as shown in SEQ ID NO. 1.

2. The *cis*-epoxysuccinate hydrolase-encoding gene of claim 1, a nucleotide sequence of the *cis*-epoxysuccinate hydrolase-encoding gene is as shown in SEQ ID NO. 1.

3. A *cis*-epoxysuccinate hydrolase-encoding gene, **characterized in that** the *cis*-epoxysuccinate hydrolase-encoding gene has a nucleotide sequence that is more than 70% homologous to a nucleotide sequence as shown in SEQ ID NO. 1.

4. The *cis*-epoxysuccinate hydrolase-encoding gene of claim 3, having nucleotide sequence that is more than 80% homologous to the nucleotide sequence as shown in SEQ ID NO. 1.

5. The *cis*-epoxysuccinate hydrolase-encoding gene of claim 4, having nucleotide sequence that is more than 90% homologous to the nucleotide sequence as shown in SEQ ID NO. 1.

6. The *cis*-epoxysuccinate hydrolase-encoding gene of claim 3, having a nucleotide sequence as shown in SEQ ID NO. 23, SEQ ID NO. 26, SEQ ID NO. 10, SEQ ID NO. 15 or SEQ ID NO. 19.

7. A polypeptide, **characterized in that** the polypeptide has an amino acid sequence as shown in SEQ ID NO. 2.

8. A polypeptide encoded by the gene of claim 2, **characterized in that** the amino acid sequence of the polypeptide is as shown in SEQ ID NO. 2.

9. A polypeptide, **characterized in that**: the polypeptide has an amino acid sequence that is derived based on the amino acid sequence as shown in SEQ ID NO. 2 through substitution, deletion or addition of amino acids, the polypeptide is more than 50% homologous to that shown in SEQ ID NO. 2, wherein the polypeptide exhibits *cis*-epoxysuccinate hydrolase activity, and the *cis*-epoxysuccinate hydrolase activity level is at least 95% of that of the complete amino acid sequence SEQ ID NO. 2.

**10.** The polypeptide of claim 9, having an amino acid sequence as shown in SEQ ID NO. 24, SEQ ID NO. 27, SEQ ID NO. 12, SEQ ID NO. 17 or SEQ ID NO. 20.

**11.** A polynucleotide, **characterized in that** it has a nucleotide sequence for encoding the polypeptide of claim 9.

**12.** The polynucleotide of claim 11, having nucleotide sequence that is more than 70% homologous with the nucleotide sequence as shown in SEQ ID NO. 1.

**13.** A recombinant expression vector or a recombinant microbial strain that is usable for expressing the polypeptide of any of claims 7-10.

**14.** A method for preparing L(+)-tartaric acid or its salts using the polypeptide of any of claims 7-11, **characterized in that** the L(+)-tartaric acid or its salts are produced by hydrolysis of *cis*-epoxysuccinic acid or salts thereof under catalysis of the said polypeptide .

**Patentansprüche**

**1.** *cis*-Epoxysuccinat-Hydrolase kodierendes Gen, **dadurch gekennzeichnet, dass**: das *cis*-Epoxysuccinat-Hydrolase kodierende Gen ein Segment einschließt, das eine Nukleotidsequenz aufweist wie in SEQ.-ID Nr 1 gezeigt.

**2.** *cis*-Epoxysuccinat-Hydrolase kodierendes Gen nach Anspruch 1, wobei eine Nukleotidsequenz des *cis*-Epoxysuccinat-Hydrolase kodierenden Gens ist wie in SEQ.-ID Nr. 1 gezeigt.

**3.** *cis*-Epoxysuccinat-Hydrolase kodierendes Gen, **dadurch gekennzeichnet, dass** das *cis*-Epoxysuccinat-Hydrolase kodierende Gen eine Nukleotidsequenz aufweist, die mehr als 70 % homolog zu einer Nukleotidsequenz ist wie in SEQ.-ID Nr. 1 gezeigt.

**4.** *cis*-Epoxysuccinat-Hydrolase kodierendes Gen nach Anspruch 3, das eine Nukleotidsequenz aufweist, die mehr als 80 % homolog zu der Nukleotidsequenz ist wie in SEQ.-ID Nr. 1 gezeigt.

**5.** *cis*-Epoxysuccinat-Hydrolase kodierendes Gen nach Anspruch 4, das eine Nukleotidsequenz aufweist, die mehr als 90 % homolog zu der Nukleotidsequenz ist wie in SEQ.-ID Nr. 1 gezeigt.

**6.** *cis*-Epoxysuccinat-Hydrolase kodierendes Gen nach Anspruch 3, das eine Nukleotidsequenz aufweist wie in SEQ.-ID Nr. 23, SEQ.-ID Nr. 26, SEQ.-ID Nr. 10, SEQ.-ID Nr. 15 oder SEQ.-ID Nr. 19 gezeigt.

**7.** Polypeptid, **dadurch gekennzeichnet, dass** das Polypeptid eine Aminosäuresequenz aufweist wie in SEQ.-ID Nr. 2 gezeigt.

**8.** Polypeptid, das von dem Gen nach Anspruch 2 kodiert wird, **dadurch gekennzeichnet, dass** die Aminosäuresequenz des Polypeptids ist wie in SEQ.-ID Nr. 2 gezeigt.

**9.** Polypeptid, **dadurch gekennzeichnet, dass**: das Polypeptid eine Aminosäuresequenz aufweist, die, basierend auf der Aminosäuresequenz wie in SEQ.-ID Nr. 2 gezeigt, durch Substitution, Deletion oder Addition von Aminosäuren abgeleitet ist, das Polypeptid mehr als 50 % homolog ist zu der, die in SEQ.-ID Nr. 2 gezeigt ist, wobei das Polypeptid *cis*-Epoxysuccinat-Hydrolase-Aktivität zeigt und die Höhe der *cis*-Epoxysuccinat-Hydrolase-Aktivität mindestens 95 % von der der vollständigen Aminosäuresequenz SEQ.-ID Nr. 2 beträgt.

**10.** Polypeptid nach Anspruch 9, das eine Aminosäuresequenz aufweist wie in SEQ.-ID Nr. 24, SEQ.-ID Nr. 27, SEQ.-ID Nr. 12, SEQ.-ID Nr. 17 oder SEQ.-ID Nr. 20 gezeigt.

**11.** Polynukleotid, **dadurch gekennzeichnet, dass** es eine Nukleotidsequenz für die Kodierung des Polypeptids nach Anspruch 9 aufweist.

**12.** Polynukleotid nach Anspruch 11, das eine Nukleotidsequenz aufweist, die mehr als 70 % homolog zu der Nukleotidsequenz ist wie in SEQ.-ID Nr. 1 gezeigt.

13. Rekombinanter Expressionsvektor oder ein rekombinanter mikrobieller Stamm, der für die Expression des Polypeptids nach einem der Ansprüche 7-10 verwendbar ist.

14. Verfahren für die Herstellung von L(+)-Weinsäure oder ihrer Salze unter Verwendung des Polypeptids nach einem der Ansprüche 7-11, **dadurch gekennzeichnet, dass** die L(+)-Weinsäure oder ihre Salze durch Hydrolyse von *cis*-Epoxybernsteinsäure oder der Salze davon unter Katalyse des Polypeptids erzeugt werden.

## Revendications

1. Gène codant pour une *cis*-époxysuccinate hydrolase, **caractérisé en ce que** le gène codant pour une *cis*-époxysuccinate hydrolase comporte un segment ayant une séquence nucléotidique telle qu'illustrée dans SEQ ID n° 1.

2. Gène codant pour une *cis*-époxysuccinate hydrolase selon la revendication 1, une séquence nucléotidique du gène codant pour une *cis*-époxysuccinate hydrolase étant telle qu'illustrée dans SEQ ID n° 1.

3. Gène codant pour une *cis*-époxysuccinate hydrolase, **caractérisé en ce que** le gène codant pour une *cis*-époxysuccinate hydrolase comporte une séquence nucléotidique qui possède plus de 70% d'homologie par rapport à une séquence nucléotidique telle qu'illustrée dans SEQ ID n° 1.

4. Gène codant pour une *cis*-époxysuccinate hydrolase selon la revendication 3, ayant une séquence nucléotidique qui possède plus de 80% d'homologie par rapport à la séquence nucléotidique telle qu'illustrée dans SEQ ID n° 1.

5. Gène codant pour une *cis*-époxysuccinate hydrolase selon la revendication 4, ayant une séquence nucléotidique qui possède plus de 90% d'homologie par rapport à la séquence nucléotidique telle qu'illustrée dans SEQ ID n° 1.

6. Gène codant pour une *cis*-époxysuccinate hydrolase selon la revendication 3, ayant une séquence nucléotidique telle qu'illustrée dans SEQ ID n° 23, SEQ ID n° 26, SEQ ID n° 10, SEQ ID n° 15 ou SEQ ID n° 19.

7. Polypeptide, **caractérisé en ce que** le polypeptide possède une séquence d'acides aminés telle qu'illustrée dans SEQ ID n° 2.

8. Polypeptide codé par le gène selon la revendication 2, **caractérisé en ce que** la séquence d'acides aminés du polypeptide est telle qu'illustrée dans SEQ ID n° 2.

9. Polypeptide, **caractérisé en ce que** le polypeptide possède une séquence d'acides aminés qui est dérivée de la séquence d'acides aminés telle qu'illustrée dans SEQ ID n° 2 par substitution, délétion ou addition d'acides aminés, le polypeptide possédant plus de 50% d'homologie par rapport à celui illustré dans SEQ ID n° 2, où le polypeptide présente une activité de *cis*-époxysuccinate hydrolase, et le taux d'activité de *cis*-époxysuccinate hydrolase constitue au moins 95% de celui de la séquence d'acides aminés complète de SEQ ID n° 2.

10. Polypeptide selon la revendication 9, ayant une séquence d'acides aminés telle qu'illustrée dans SEQ ID n° 24, SEQ ID n° 27, SEQ ID n° 12, SEQ ID n° 17 ou SEQ ID n° 20.

11. Polynucléotide, **caractérisé en ce qu'**il possède une séquence nucléotidique destinée à coder le polypeptide selon la revendication 9.

12. Polynucléotide selon la revendication 11, ayant une séquence nucléotidique qui possède plus de 70% d'homologie par rapport à la séquence nucléotidique telle qu'illustrée dans SEQ ID n° 1.

13. Vecteur d'expression recombiné ou souche microbienne recombinée, qui est utilisable pour l'expression du polypeptide selon l'une quelconque des revendications 7-10.

14. Méthode de préparation d'acide L(+)tartrique ou de ses sels en utilisant le polypeptide selon l'une quelconque des revendications 7-11, **caractérisée en ce que** l'acide L(+)tartrique ou ses sels sont produits par l'hydrolyse d'acide *cis*-époxysuccinique ou de ses sels via une catalyse par ledit polypeptide.

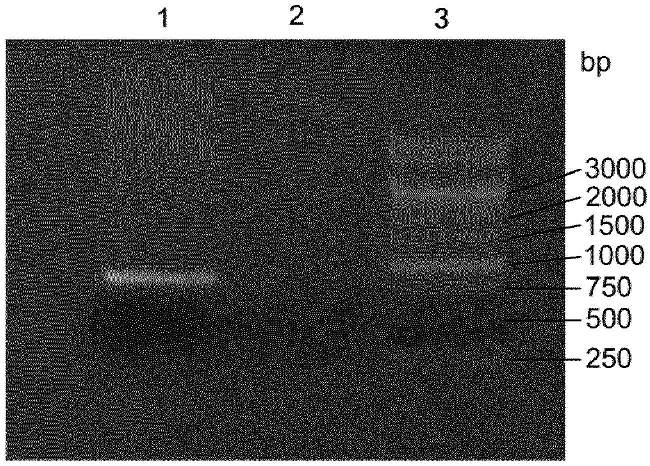


Figure 1

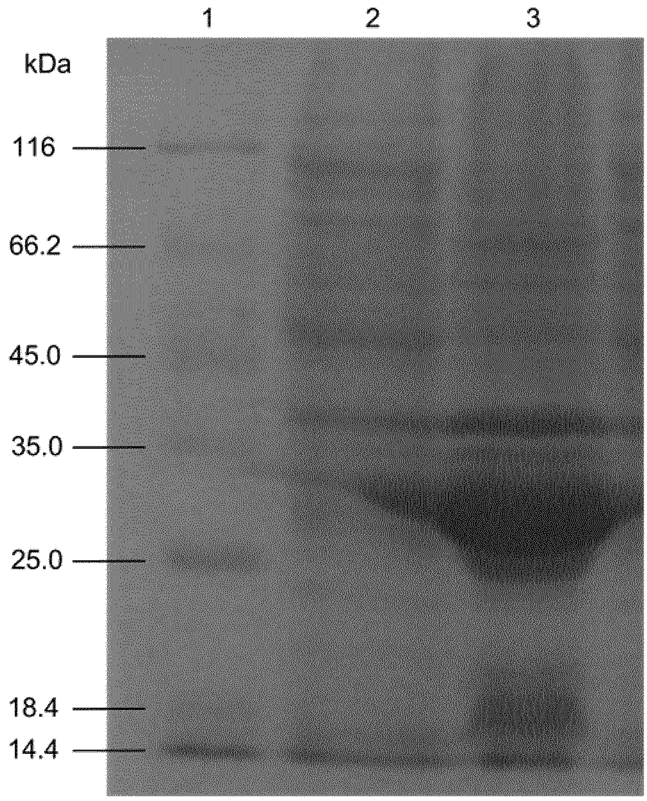


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4028185 A
- US 4013509 A
- US 4011135 A